(19)

**Europäisches Patentamt**
**European Patent Office**
**Office européen des brevets**

(11)  **EP 2 986 641 B1**

(12)  # EUROPEAN PATENT SPECIFICATION

(45) Date of publication and mention
of the grant of the patent:
**07.11.2018 Bulletin 2018/45**

(51) Int Cl.:
*C07K 16/26* (2006.01)　　*A61P 5/00* (2006.01)
*A61K 39/395* (2006.01)　*A61K 39/00* (2006.01)
*A61K 38/22* (2006.01)　　*G01N 33/74* (2006.01)

(21) Application number: **14718566.4**

(22) Date of filing: **16.04.2014**

(86) International application number:
**PCT/EP2014/057704**

(87) International publication number:
**WO 2014/170362 (23.10.2014 Gazette 2014/43)**

(54) **ANTI-GHRELIN ANTIBODIES AND USES THEREOF**

ANTI-GHRELIN-ANTIKÖRPER UND VERWENDUNGEN DAVON

ANTICORPS ANTI-GHRELIN ET LEURS UTILISATIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.04.2013 EP 13305495**

(43) Date of publication of application:
**24.02.2016 Bulletin 2016/08**

(73) Proprietors:
• **Institut National de la Santé et de la
Recherche Médicale (INSERM)
75013 Paris (FR)**
• **Université de Rouen
76130 Mont-Saint-Aignan (FR)**
• **Kagoshima University
Kagoshima-shi, Kagoshima 890-8580 (JP)**
• **Centre Hospitalier Universitaire de Rouen
76000 Rouen (FR)**

(72) Inventors:
• **FETISSOV, Serguei
76183 Rouen (FR)**
• **LEGRAND, Romain
76183 Rouen (FR)**

• **DECHELOTTE, Pierre
76183 Rouen (FR)**
• **INUI, Akio
Kagoshima
Kagoshima 890-8520 (JP)**
• **ASAKAWA, Akihiro
Kagoshima
Kagoshima 890-8544 (JP)**
• **TAKAGI, Kuniko
Kagoshima
Kagoshima 890-8520 (JP)**

(74) Representative: **Icosa
83 avenue Denfert-Rochereau
75014 Paris (FR)**

(56) References cited:
**WO-A1-2007/099346　　WO-A2-2005/016951
WO-A2-2010/079428**

• **E. ZORRILLA ET AL.: "Vaccinatin against weight
gain.", PROCEEDINGS OF THE NATIONAL
ACADEMY OF SCIENCES OF THE USA, vol. 103,
no. 35, 28 September 2006 (2006-09-28), pages
13226-13231, XP002415416, Washington, DC,
USA cited in the application**

EP 2 986 641 B1

SEQUENCE LISTING

<110> INSERM

<120> Anti-ghrelin antibodies and uses thereof

<130> BET 14P0839

<150> EP 13305495.7
<151> 2013-04-16

<160> 1

<170> PatentIn version 3.5

<210> 1
<211> 28
<212> PRT
<213> Homo sapiens

<400> 1

```
Gly Ser Ser Phe Leu Ser Pro Glu His Gln Arg Val Gln Gln Arg Lys
1               5               10                  15

Glu Ser Lys Lys Pro Pro Ala Lys Leu Gln Pro Arg
            20                  25
```

**Description**

**Field of the invention**

The present invention relates to antibodies which bind human ghrelin and their use in methods of treating or preventing reduced appetite. The invention further relates to diagnostic tools for determining whether an individual is likely to respond to a method of treating or preventing reduced appetite.

**Background to the invention**

Obesity is commonly accompanied by increased hunger and food intake; however, the plasma concentrations of ghrelin, the principal hunger hormone, remain normal in obese individuals. This indicates that in obesity, ghrelin may transmit even more powerful hunger signals to the central circuitries regulating appetite (Wu et al., 2012. Nature. 483(7391):594-597). In fact, ghrelin administration has been shown to stimulate food intake more efficiently in obese than in lean humans, but the underlying mechanisms remain unknown (Druce et al., 2005. Int J Obes Relat Metab Disord. 29(9):1130-1136). Studies assaying total ghrelin in plasma have reported its lower levels in obese subjects, but as later clarified, this decrease was due to the low levels of des-acyl-ghrelin, while acyl-ghrelin remained normal, suggesting decreased degradation of acyl-ghrelin in the obese subjects. Ghrelin is secreted by the stomach as an acylated peptide which then degrades to des-acyl ghrelin having no orexigenic effects (Gutierrez et al., 2008. Proc. Natl Acad. Sci. 105(17):6320-6325; Asakawa et al, 2005. Gut. 54(1):18-24).

Recently, ghrelin-reactive Ig, *i.e.,* autoantibodies (autoAbs), have been identified in healthy subjects and in anorexia nervosa (AN) patients (Fetissov et al., 2008. Nutrition. 24(4):348-359; Terashi et al., 2011. Nutrition. 27(4):407-413).

Furthermore, studies in rodents showed that stimulation of ghrelin secretion leads to simultaneous increase of ghrelin-reactive IgG (Gallas & Fetissov, 2011. Peptides. 32(11):2283-2289), and that raising antibodies against ghrelin may lead to decreased body weight in immunized animals (Zorrilla et al., 2006. Proc Natl Acad Sci U S A. 103(35):13226-13231). In this respect, International patent application WO2007099346 (Cambridge Antibody Technology Limited) discloses human antibodies, namely IgG clones CP1A1, which bind to acyl-ghrelin (with a $K_D$ of 708 pM, a $k_a$ of $4.73x10^5$ $M^{-1}s^{-1}$ and a $k_d$ of $3.35x10^{-4}$ $s^{-1}$) and block its interaction with its receptor, namely GHSR-1a. Clinical indications in which such anti-ghrelin antibody may be used include obesity and obesity-related conditions.

Consequently, it seemed possible that changes of properties of the naturally present ghrelin-reactive IgG may influence the levels and effects of ghrelin, including its enhanced orexigenic action in obesity.

**Description of the invention**

The present Inventors have shown that ghrelin-reactive immunoglobulins (Ig) are responsible for ghrelin's enhanced orexigenic effects in obesity. Using surface plasmon resonance (SPR) technology, affinity kinetics between ghrelin and plasma IgG were measured and it was found that IgGs from obese subjects, as compared to non-obese and anorectic subjects, display increased affinities for ghrelin remaining in the micromolar range. Furthermore, in satiated rats, co-administration of ghrelin together with IgG from obese, anorectic or controls subjects led to increased food intake only in rats receiving IgG from obese humans.

Specifically, the Inventors showed *in vivo* that rats receiving ghrelin in combination with IgG antibody displayed an increased food intake as compared to control groups, but only if said IgG antibody derived from obese individuals. The Inventors further showed that said obese-derived IgG antibodies displayed modified affinity kinetics towards their antigen, ghrelin, in comparison to antibodies not affecting food intake when administered together with ghrelin.

The Inventors found that the equilibrium dissociation constant ($K_D$) values of ghrelin-reactive IgG were in the micromolar range (between $10^{-7}$ M and $10^{-6}$ M) and that the antibodies were distinguished from the ineffective antibodies mainly in their higher association rate constant ($k_a$). The mean dissociation rate constant ($k_d$) values in obese individuals' IgG were not significantly different from controls.

The Inventor's findings reveal that ghrelin can be transported by plasma IgG preserving its biological activity and that in obesity, changes of the affinity kinetics of IgG for ghrelin may underlie enhanced hunger signal.

Broadly, the invention relates to a composition comprising antibodies with affinities for ghrelin comparable to the affinities identified for IgGs isolated from obese individuals, and the use of said antibodies in combination with ghrelin to treat people who suffer from reduced appetite. The invention further relates to diagnostic methods which help to identify patients susceptible for said appetite stimulating therapy.

Thus, the invention relates to a composition comprising a ghrelin binding antibody or fragment thereof that specifically binds human ghrelin and that:

i) exhibits an equilibrium dissociation constant ($K_D$) at 25°C in the range from $10^{-7}$ M to $10^{-6}$ M for human ghrelin;
ii) exhibits an association rate constant ($k_a$) at 25°C in the range from $0.5 \times 10^4$ to $0.5 \times 10^5$ $M^{-1}s^{-1}$ for human ghrelin; and
iii) exhibits a dissociation rate constant ($k_d$) at 25°C in the range from $10^{-3}$ to $10^{-2}s^{-1}$ for human ghrelin;

wherein said $K_D$, $k_a$, and $k_d$ values are determined by surface plasmon resonance; and wherein optionally

iv) the ghrelin antibody does not inhibit human ghrelin to bind and activate hGHSR1a.

The antibodies can be, for example, IgM, IgD, IgG, IgA and IgE antibodies.
In some embodiments, the antibody is isolated from an obese individual, wherein the obese individual has a BMI > 30.
In some embodiments, the antibody is a monoclonal antibody.
In some embodiments, the monoclonal antibody is humanized.
The term antibody includes antibody fragments, including Fv, Fab, F(ab')$_2$, Fab', dsFv, (dsFv)$_2$, scFv, sc(Fv)$_2$, diabodies and VHH.
The composition may further comprise ghrelin, a derivative, a variant or fragment thereof that retains the ability to bind hGHSR1.
The composition may be a pharmaceutical composition, further comprising a pharmaceutically acceptable carrier, diluent, or excipient.
In some embodiments, the composition is for use as a medicament.
Said composition may be used in enhancing ghrelin pharmacological effects.
In some embodiments, the composition is for use in a method of treatment or prevention of a disease or disorder related to reduced appetite.
Also provided is a method of treating or preventing a disease or disorder related to reduced appetite, said method preferably comprising administering to an individual in need thereof a composition of the invention.
Also provided is the use of said composition for the manufacture of a medicament for the treatment or prevention of a disease or disorder related to reduced appetite.
The disease or disorder may include anorexia, anorexia nervosa, bulimia, cachexia, lipodystrophia and wasting diseases.
The composition may be used, for example, to stimulate appetite, wherein the reduced appetite is due to anorexia, anorexia nervosa, bulimia, cachexia, lipodystrophia or wasting diseases.
"Cachexia" may be a wasting syndrome associated with loss of weight and/or muscle atrophy and/or fatigue, weakness, and significant loss of appetite which may be caused by cancer, AIDS, chronic obstructive lung disease, multiple sclerosis, congestive heart failure, tuberculosis, familial amyloid polyneuropathy, mercury poisoning (acrodynia) and hormonal deficiency.
In some embodiments, cachexia is caused by cancer, and is thus termed "cancer cachexia".
The invention further provides a cosmetic non-therapeutic method of preventing reduced appetite in an individual, comprising administering to said individual an effective amount of said composition.
The invention further provides a cosmetic non-therapeutic method of altering, in particular increasing, food intake and/or the weight of an individual, comprising administering to said individual an effective amount of said composition.
The invention further provides a diagnostic method of selecting patients suffering from reduced appetite as likely to respond to a method of treating or preventing reduced appetite comprising administering to said patients a composition according to any one of claims 1 to 8, wherein said diagnostic method comprises:

i) providing antibodies from said subject, optionally by isolating antibodies from a blood, plasma or serum sample of the subject;
ii) measuring the affinity of said antibodies to ghrelin;
iii) selecting the patient for an appetite stimulating therapy where the dissociation rate constant of at least one of said antibodies is $k_d > 0.0023$ $s^{-1}$.

In one embodiment, said subject may suffer from reduced appetite.
In a further embodiment, said subject may suffer from loss of appetite.
In a further embodiment at least one of said antibodies has at least one of the following:

i) an equilibrium dissociation constant ($K_D$) at 25°C in the range from $10^{-7}$M to $10^{-6}$ M for human ghrelin; and/or
ii) an association rate constant ($k_a$) at 25°C in the range from $0.5*10^4$ to $0.5*10^5$ $M^{-1}s^{-1}$ for human ghrelin; and/or
iii) a dissociation rate constant ($k_d$) at 25°C in the range from $10^{-3}$ to $10^{-2}s^{-1}$ for human ghrelin, wherein said $K_D$, $k_a$,

$k_d$ values are determined by surface plasmon resonance.

The diagnostic methods of the invention are preferably *in vitro* or *ex vivo* methods.

"Ghrelin" was identified originally as an endogenous ligand for the growth hormone secretagogue receptor (GHSR) in rat stomach and later found to be a major regulator of appetite, food intake and energy homeostasis. Similar to many other bioactive peptides, ghrelin probably acts as a hormone, a paracrine substance and as a neurotransmitter.

In humans, ghrelin comprises a chain of 28 amino acids and has the amino acid sequence GSSFLSPEHQRVQQRKESKKPPAKLQPR (SEQ ID NO: 1) corresponding to the amino acids 24 to 51 of the human ghrelin sequence (AAQ89412.1, GenBank). Ghrelin derives from the precursor preproghrelin which is cleaved to yield proghrelin, which is then acylated at the serine at amino acid position three ($Ser^3$) by ghrelin O-acyltransferase to yield octanoyl ghrelin and decanoyl ghrelin. "Ghrelin" as used herein includes acyl and des-acyl ghrelin.

Only octanoyl ghrelin is able to bind and activate the growth hormone secretagogue receptor (GHSR1a receptor) in the hypothalamus pituitary and other tissues, causing appetite stimulation and resulting in release of growth hormone (GH). Unacylated ghrelin (des-acyl ghrelin) is also present in plasma but its function is controversial.

The main site for ghrelin production is the stomach, where the peptide is found in classical endocrine cells in the gastric mucosa principally secreted from X/A-like cells within gastric oxyntic glands, but ghrelin also occurs in the hypothalamus, pituitary, adrenal gland, thyroid, breast, ovary, placenta, fallopian tube, testis, prostate, liver, gall bladder, pancreas, fat tissue, human lymphocytes, spleen, kidney, lung, skeletal muscle, myocardium, vein and skin.

Ghrelin is secreted in the pre-meal situation which results in a sharp, short-lived surge in plasma levels of ghrelin before the meal and starting 1-2 hours before and lasting a short while after initiation of the meal and falls according to the calories ingested.

Since ghrelin is the only known peripherally produced orexigenic (appetite promoting) substance, it is believed that the increase in plasma levels of ghrelin is crucial for the initiation of a meal.

Persons with Prader-Willi syndrome, a genetic disorder that causes severe obesity with uncontrollable appetite, have extremely high levels of ghrelin. These observations indicate that ghrelin plays a key role in motivating feeding.

Experiments have shown that both central and peripheral administration of ghrelin increase food intake and body weight along with a reduction in fat utilization in rodents. Negative correlations between circulating ghrelin levels and body mass index are found in human. Surprisingly, fasting plasma levels of ghrelin are reported to be high in patients with anorexia nervosa and subjects with diet-induced weight loss. In contrast, obese subjects show a less marked drop in plasma ghrelin after meal ingestion. In patients with heart failure, increased levels of plasma ghrelin are reported in cachectic patients when compared with non-cachectic patients.

Therefore, ghrelin concentration in plasma cannot be the only factor regulating hunger signaling. The inventors have shown that binding kinetics of auto-immune antibodies directed against ghrelin may play a crucial role in hunger signaling.

Secretion of ghrelin is inhibited by insulin, growth hormone (somatotropin), leptin, glucose, glucagon, and fatty acids. Secretion is stimulated by insulin-like growth factor-1 and muscarinic agonists. In the bloodstream, acyl ghrelin is deacylated by butyrylcholinesterase and platelet activating factor acetylhydrolase. Other enzymes may also deacylate acyl ghrelin.

Ghrelin, according to the invention, is known for its appetite stimulating function due to its binding and activation of hGHSR1a resulting in the secretion of growth hormone.

The growth hormone secretagogue receptor "GHSR1a" was initially described as an orphan receptor, activated by synthetic peptidyl growth hormone secretagogues (GHS), such as herexalin, growth hormone releasing peptides (GHRP1, GHRP2 and GHRP6) and the non-peptidyl ligand, MK-0677, which were all shown to stimulate the release of growth hormone (GH) from the pituitary gland. Shortly thereafter, ghrelin was identified as the endogenous ligand for the GHSR1a receptor. The GHSR1a receptor belongs, with its 366 amino acids, seven transmembrane regions and molecular mass of approximately 41 kDa, to class 1 of G-protein coupled receptors (GPCR). GHSR1a is expressed in both the peripheric and central nervous system and, when activated by ghrelin, mediates a multitude of biological activities, including the secretion of GH as well as the stimulation of appetite and food intake, maintaining the body's energy homeostasis. Expression of the GHSR1a receptor in the extra-hypothalamic neurocircuitry, regulating this non-homeostatic feeding, including the ventral tegmental area (VTA), nucleus accumbens (NAcc), hippocampus and amygdala, is in line with ghrelin's role in the hedonic aspects of food intake.

The pathway activated by binding of ghrelin to GHSR1a regulates the activation of the downstream mitogen-activated protein kinase Akt, nitric oxide synthase and AMPK cascades in different cellular systems.

Ghrelin binding to and activation of GHSR1a results in the secretion of growth hormone and/or an increase in $Ca^{2+}$ influx into the cell.

"Growth hormone" is a hormone important for growth, cell reproduction and regeneration in individuals. Binding of ghrelin to the growth hormone secretagogue receptor is important for its appetite stimulating function, as described above.

As used herein, "ghrelin pharmacological effects" are biochemical and/or physiological effects of ghrelin on the cell, tissue, organ, or organism. Said ghrelin pharmacological effects includes the effects on food intake and growth hormone release but ghrelin has also important vascular and metabolic actions.

In one embodiment, ghrelin pharmacological effects are associated with having orexigenic, adipogenic and somatotrophic properties, increasing food intake and body weight.

Additionally, ghrelin pharmacological effects include NO production, preferably using a signaling pathway that involves GHSR1a, PI 3-kinase, Akt and eNOS, pathways shared in common with the insulin receptor.

Therefore, in some embodiments, enhancing ghrelin pharmacological effects may be relevant to treat diabetes and related diseases. Enhancing ghrelin pharmacological effects may also be used to lower peripheral resistance, improve contractility and cardiac output. Ghrelin also has anti-inflammatory and antiapoptotic effects both *in vivo* and *in vitro.*

Derivatives, fragments or variants of ghrelin retaining the ability to bind and activate hGHSR1a are also within the scope of the invention.

"Variants" of ghrelin may be naturally occurring variants, such as splice variants, alleles and isoforms, or they may be produced by recombinant means. Variations in amino acid sequence may be introduced by substitution, deletion or insertion of one or more codons into the nucleic acid sequence encoding the protein that results in a change in the amino acid sequence of the protein. Optionally the variation is by substitution of 1, 2, 3, 4, 5, 6, 7, 8, 9, 10, 11, 12, 13, 14, 15, 16, 17, 18, 19, 20 or more amino acids with any other amino acid in the protein. Additionally or alternatively, the variation may be by addition or deletion of 1, 2, 3, 4, 5, 6, 7, 8, 9, 10, 11, 12, 13, 14, 15, 16, 17, 18, 19, 20, 40 or 50 or more amino acids within the protein.

"Fragments" of ghrelin, derivatives and variants disclosed herein are also encompassed by the invention. Such fragments may be truncated at the N-terminus or C-terminus, or may lack internal residues, for example, when compared with a full-length protein. Certain fragments lack amino acid residues that are not essential for enzymatic activity. Preferably, said fragments are at least about 5, 6, 7, 8, 9, 10, 11, 12, 13, 14, 15, 16, 17, 18, 19, 20, 21, 22, 23, 24, 25, 26, 27, 28 or more amino acids in length. Furthermore, "derivatives" comprise C- or N-terminally tagged fragments or variants of ghrelin.

Ghrelin variants may include proteins that have at least about 80% amino acid sequence identity with a polypeptide sequence disclosed herein. Preferably, a variant protein will have at least about 50%, 55%, 60%, 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% amino acid sequence identity to a full-length polypeptide sequence or a fragment of a polypeptide sequence as disclosed herein. Amino acid sequence identity is defined as the percentage of amino acid residues in the variant sequence that are identical with the amino acid residues in the reference sequence, after aligning the sequences and introducing gaps, if necessary, to achieve the maximum percent sequence identity, and not considering any conservative substitutions as part of the sequence identity. Sequence identity may be determined over the full length of the variant sequence, the full length of the reference sequence, or both. By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% (5 of 100) of the amino acid residues in the subject sequence may be inserted, deleted, or substituted with another amino acid.

In the context of the present application, the percentage of identity is calculated using a global alignment (*i.e.,* the two sequences are compared over their entire length). Methods for comparing the identity of two or more sequences are well known in the art. The « Needle » program, which uses the Needleman-Wunsch global alignment algorithm (Needleman & Wunsch, 1970. J Mol Biol. 48(3):443-453) to find the optimum alignment (including gaps) of two sequences when considering their entire length, may for example be used. The needle program is, for example, available on the ebi.ac.uk world wide web site. The percentage of identity in accordance with the invention is preferably calculated using the EMBOSS Needle (global) program with a "Gap Open" parameter equal to 10.0, a "Gap Extend" parameter equal to 0.5, and a Blosum62 matrix.

Proteins consisting of an amino acid sequence "at least 80%, 85%, 90%, 95%, 96%, 97%, 98% or 99% identical" to a reference sequence may comprise mutations such as deletions, insertions and/or substitutions compared to the reference sequence. In case of substitutions, the protein consisting of an amino acid sequence at least 80%, 85%, 90%, 95%, 96%, 97%, 98% or 99% identical to a reference sequence may correspond to a homologous sequence derived from another species than the reference sequence. Amino acid substitutions may be conservative or non-conservative. Preferably, substitutions are conservative substitutions, in which one amino acid is substituted for another amino acid with similar structural and/or chemical properties. The substitution preferably corresponds to a conservative substitution as indicated in the table below.

| Conservative substitutions | Type of Amino Acid |
| --- | --- |
| Ala, Val, Leu, Ile, Met, Pro, Phe, Trp | Amino acids with aliphatic hydrophobic side chains |
| Ser, Tyr, Asn, Gln, Cys | Amino acids with uncharged but polar side chains |
| Asp, Glu | Amino acids with acidic side chains |
| Lys, Arg, His | Amino acids with basic side chains |
| Gly | Neutral side chain |

Derivatives, variants and fragments of ghrelin according to the invention maintain the ability to agonize hGHSR1a and therefore have an appetite stimulating function. It is known that short peptides encompassing the first 4 or 5 residues of ghrelin retain the ability to activate functionally hGHSR1a. Specifically, the Gly-Ser-Ser(n-octanoyl)-Phe segment is the "active core" required for agonist potency and function (Bednarek et al., 2000. J Med Chem. 43(23):4370-4376).

An "antibody" or "immunoglobulin" (Ig) may be a natural or conventional antibody in which two heavy chains are linked to each other by disulfide bonds and each heavy chain is linked to a light chain by a disulfide bond. There are two types of light chain: lambda (l) and kappa (k). There are five main heavy chain classes (or isotopes) which determine the functional activity of an antibody molecule: IgM, IgD, IgG, IgA and IgE. Each chain contains distinct sequence domains. The light chain includes two domains or regions, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) influence the overall domain structure and hence the combining site. "Complementarity Determining Regions" or CDRs refer to amino acid sequences which together define the binding affinity and specificity of the natural Fv region of a native immunoglobulin binding site. The light and heavy chains of an immunoglobulin each have three CDRs, designated CDR1-L, CDR2-L, CDR3-L and CDR1-H, CDR2-H, CDR3-H, respectively. A conventional antibody/antigen-binding site, therefore, includes six CDRs, comprising the CDR set from each of a heavy and a light chain V region.

"Framework Regions" (FRs) refer to amino acid sequences interposed between CDRs, *i.e.,* to those portions of immunoglobulin light and heavy chain variable regions that are relatively conserved among different immunoglobulins in a single species. The light and heavy chains of an immunoglobulin each have four FRs, designated FR1-L, FR2-L, FR3-L, FR4-L, and FR1-H, FR2-H, FR3-H, FR4-H, respectively.

As used herein, a "human framework region" is a framework region that is substantially identical (about 85%, or more, in particular 90%, 95%, 97%, 99% or 100%) to the framework region of a naturally occurring human antibody.

As used herein, the term "antibody" denotes conventional antibodies and fragments thereof, as well as single domain antibodies and fragments thereof, in particular variable heavy chain of single domain antibodies, and chimeric, humanized, bispecific or multispecific antibodies.

As used herein, antibody or immunoglobulin also includes "single domain antibodies" which have been more recently described and which are antibodies whose complementary determining regions are part of a single domain polypeptide. Examples of single domain antibodies include heavy chain antibodies, antibodies naturally devoid of light chains, single domain antibodies derived from conventional four-chain antibodies, engineered single domain antibodies. Single domain antibodies may be derived from any species including, but not limited to, mouse, human, camel, llama, goat, rabbit and bovine. Single domain antibodies may be naturally occurring single domain antibodies known as heavy chain antibody devoid of light chains. In particular, *Camelidae* species, for example camel, dromedary, llama, alpaca and guanaco, produce heavy chain antibodies naturally devoid of light chain. Camelid heavy chain antibodies also lack the CH1 domain.

The variable heavy chain of these single domain antibodies devoid of light chains are known in the art as "VHH" or "nanobody". Similar to conventional VH domains, VHHs contain four FRs and three CDRs. Nanobodies have advantages over conventional antibodies: they are about ten times smaller than IgG molecules, and as a consequence properly folded functional nanobodies can be produced by *in vitro* expression while achieving high yield. Furthermore, nanobodies are very stable, and resistant to the action of proteases. The properties and production of nanobodies have been reviewed by Harmsen & De Haard (**2007**. *Appl Microbiol Biotechnol.* **77(1):**13-22).

The antibody of the invention may be a polyclonal antibody, in particular a polyclonal antibody isolated from an obese individual, or a monoclonal antibody. Preferably, the antibody is a human antibody.

The antibody may be a IgM, IgD, IgG, IgA and IgE antibody, in particular an IgG antibody. The antibody may be a monoclonal antibody. Said monoclonal antibody may be humanized. In another example, the antibody may be a fragment selected from the group consisting of Fv, Fab, F(ab')$_2$, Fab', dsFv, (dsFv)2, scFv, sc(Fv)2, diabodies and VHH. The term "monoclonal antibody" or "mAb" as used herein refers to an antibody molecule of a single amino acid composition that is directed against a specific antigen, and is not to be construed as requiring production of the antibody by any particular method. A monoclonal antibody may be produced by a single clone of B cells or hybridoma, but may also be recombinant, *i.e.,* produced by protein engineering.

The term "chimeric antibody" refers to an engineered antibody which in its broadest sense contains one or more regions from one antibody and one or more regions from one or more other antibody(ies). In particular, a chimeric antibody comprises a VH domain and a VL domain of an antibody derived from a non-human animal, in association with a CH domain and a CL domain of another antibody, in particular a human antibody. As the non-human animal, any animal such as mouse, rat, hamster, rabbit or the like can be used. A chimeric antibody may also denote a multispecific antibody having specificity for at least two different antigens. In an embodiment, a chimeric antibody has variable domains of mouse origin and constant domains of human origin.

The term "humanised antibody" refers to an antibody which is initially wholly or partially of non-human origin and which has been modified to replace certain amino acids, in particular in the framework regions of the heavy and light chains, in order to avoid or minimize an immune response in humans. The constant domains of a humanized antibody are most of the time human CH and CL domains. In an embodiment, a humanized antibody has constant domains of human origin.

"Fragments" of (conventional) antibodies comprise a portion of an intact antibody, in particular the antigen binding region or variable region of the intact antibody. Examples of antibody fragments include Fv, Fab, F(ab')$_2$, Fab', dsFv, (dsFv)2, scFv, sc(Fv)2, diabodies, bispecific and multispecific antibodies formed from antibody fragments. A fragment of a conventional antibody may also be a single domain antibody, such as a heavy chain antibody or VHH.

The term "Fab" denotes an antibody fragment having a molecular weight of about 50,000 and antigen binding activity, in which about a half of the N-terminal side of H chain and the entire L chain, among fragments obtained by treating IgG with a protease, papain, are bound together through a disulfide bond.

The term "F(ab')$_2$" refers to an antibody fragment having a molecular weight of about 100,000 and antigen binding activity, which is slightly larger than the Fab bound via a disulfide bond of the hinge region, among fragments obtained by treating IgG with a protease, pepsin.

The term "Fab'" refers to an antibody fragment having a molecular weight of about 50,000 and antigen binding activity, which is obtained by cutting a disulfide bond of the hinge region of the F(ab')$_2$.

A single chain Fv ("scFv") polypeptide is a covalently linked VH::VL heterodimer which is usually expressed from a gene fusion including VH and VL encoding genes linked by a peptide-encoding linker. The human scFv fragment of the invention includes CDRs that are held in appropriate conformation, in particular by using gene recombination techniques. Divalent and multivalent antibody fragments can form either spontaneously by association of monovalent scFvs, or can be generated by coupling monovalent scFvs by a peptide linker, such as divalent sc(Fv)$_2$.

"dsFv" is a VH::VL heterodimer stabilized by a disulphide bond.

"(dsFv)2" denotes two dsFv coupled by a peptide linker.

The term "bispecific antibody" or "BsAb" denotes an antibody which combines the antigen-binding sites of two antibodies within a single molecule. Thus, BsAbs are able to bind two different antigens simultaneously. Genetic engineering has been used with increasing frequency to design, modify, and produce antibodies or antibody derivatives with a desired set of binding properties and effector functions as described for instance in EP 2 050 764 A1.

The term "multispecific antibody" denotes an antibody which combines the antigen-binding sites of two or more antibodies within a single molecule.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

By "purified" and "isolated" it is meant, when referring to a polypeptide (e.g., the antibody of the invention) or a nucleotide sequence, that the indicated molecule is present in the substantial absence of other biological macromolecules, optionally of the same type, or other molecules with which the polypeptide is associated or found in its natural environment. The term "purified" as used herein, in particular, means the polypeptide represents at least 75%, 80%, 85%, 90%, 95%, or 98% by weight, preferably at least 80% of biological macromolecules, optionally of the same type.

An "isolated" nucleic acid molecule which encodes a particular polypeptide refers to a nucleic acid molecule which is substantially free of other nucleic acid molecules that do not encode the subject polypeptide; however, the molecule may include some additional bases or moieties which do not deleteriously affect the basic characteristics of the composition.

The "body mass index" is defined as the individual's body mass divided by the square of their height. The formulae universally used in medicine produce a unit of measure of $kg/m^2$.

"Obese" individuals as used herein preferably have a BMI > 30 and "anorexic" individuals have a BMI < 17.5.

Preferably, the binding of the antibody of the invention to ghrelin does not inhibit the binding of ghrelin to its receptor. Therefore, the affinity of the antibody to ghrelin is preferably less strong than the affinity of ghrelin to its receptor GHSR1a. "Affinity" describes the strength of binding two compounds, for example, between a receptor and its ligand or between an antibody and its antigen. The "binding affinity" describes the strength with which a compound binds to another compound. Affinity and binding affinity can be used herein interchangeably.

The interactions between compounds upon a binding event are unique and binding affinities can be used for identification of interacting compounds.

"Binding" of a compound or ligand occurs by intermolecular forces, such as ionic bonds, hydrogen bonds and van der Waals forces. The docking (association) between two compounds such as a ligand and its target molecule is reversible in biological systems.

The binding of two compounds can be described by kinetic constants and affinity constants.

The "kinetics" of a reaction describes the time dependency of the binding event. Therefore, it comprises information about

a) how fast a compound binds to or associates with another compound, wherein the velocity of that reaction is described with an association rate constant ($k_a$);

b) and how fast a compound dissociates from another compound, wherein the velocity of that reaction is described with the dissociation rate constant ($k_d$).

The equation for two compounds (A and B) binding each other (AB) is given by the equation (1):

$$A + B \underset{k_d}{\overset{k_a}{\rightleftarrows}} AB \quad (1)$$

The "rate of association" of said reaction is the number of binding events per unit of time and equals $[A] \times [B] \times k_a$, wherein $k_a$ is the "association rate constant" ($k_a$), also known in literature as "$k_{on}$" with the unit $M^{-1}s^{-1}$.

Once binding has occurred, the compounds remain bound together for a random amount of time. The "rate of dissociation" is the number of dissociation events per unit time and equals $[AB] \times k_d$. The probability of dissociation is the same at every instant of time. Equilibrium is reached when the rate of formation of new AB complexes equals the rate at which existing AB complexes dissociate. By definition the equilibrium can be defined by equation (2):

$$[A] \times [B] \times k_a = [AB] \times k_d \quad (2)$$

This equation can be rearranged to define another constant describing the equilibrium of the reaction nominated as the equilibrium dissociation constant ($K_D$).

The equilibrium dissociation constant ($K_D$) depends on temperature and partial pressure of the reaction mixture comprising the two compounds A and B is defined as:

$$\frac{[A] \times [B]}{[AB]} = \frac{K_d}{k_a} \quad (3)$$

The smaller the dissociation constant, the more tightly bound the compound is, or the higher the affinity between the two compounds. For example, a ligand with a nanomolar (nM) dissociation constant binds more tightly to a particular receptor than a ligand with a micromolar ($\mu$M) dissociation constant.

The Inventors found that ghrelin reactive IgGs obtained from study objects have an affinity to ghrelin in the micromolar range ($\mu$M). They are therefore well-positioned to mediate peptide transport because they will not compete with the nano- to pico-molar affinity of interaction between ghrelin and its receptor. The role of the ghrelin-reactive IgG in the individuals may be the protection of ghrelin from degradation. Ghrelin becomes degraded by hydrolyzing enzymes that deacylate ghrelin and have generally an affinity in the millimolar range for their substrates.

Thus, the slight increase in affinity of ghrelin-reactive IgG found in obesity might improve their properties as ghrelin carriers/protectors, while not antagonizing ghrelin receptor binding.

Antibodies of the invention thus preferably have affinities to ghrelin corresponding to the affinity of IgGs isolated from obese individuals and should preferably not inhibit the binding of ghrelin to the GHSR1a receptor; this can be deduced from kinetic values measured for the antibodies of the invention.

The antibodies of the invention are preferably not high-affinity ghrelin neutralizing antibodies, as produced by immunization, which were shown to reduce food intake after their peripheral administration in mice. Those competing antibodies display affinities in the picomolar range.

The aforementioned kinetics can be observed or measured using well known techniques including, but not limited to, ELISA, competitive ELISA, BIAcore™ or surface plasmon resonance analysis.

In some embodiments, the antibody of the invention exhibits the equilibrium dissociation constant ($K_D$) at 25°C in the range from $10^{-7}$ M to $10^{-6}$ M for human ghrelin, an association rate constant ($k_a$) at 25°C in the range from $0.5 \times 10^4$ to $0.5 \times 10^5$ $M^{-1}s^{-1}$ for human ghrelin, and a dissociation rate constant ($k_d$) at 25°C in the range from $10^{-3}$ to $10^{-2}$ $s^{-1}$ for human ghrelin, wherein said $K_D$, $k_a$, $k_d$ values are determined by surface plasmon resonance. For example, the equilibrium dissociation constant ($K_D$) at 25°C may be at least $10^{-7}$ M, preferably at least $1 \times 10^{-7}$, $1.5 \times 10^{-7}$, $2 \times 10^{-7}$, $2.5 \times 10^{-7}$, $3 \times 10^{-7}$, $3.5 \times 10^{-7}$, $4 \times 10^{-7}$, $4.5 \times 10^{-7}$, $5 \times 10^{-7}$ M for human ghrelin. The dissociation constant ($K_D$) at 25°C may be below $10^{-6}$ M, preferably below $9.5 \times 10^{-6}$, $9 \times 10^{-6}$, $8.5 \times 10^{-6}$, $8.0 \times 10^{-6}$, $7.5 \times 10^{-6}$, $7 \times 10^{-6}$, $6.5 \times 10^{-6}$, $6 \times 10^{-6}$, $5.5 \times 10^{-6}$ M for human ghrelin.

For example, the rate constant ($k_a$) at 25°C may be at least $0.5 \times 10^4$ $M^{-1}s^{-1}$, preferably at least $0.5 \times 10^4$, $0.55 \times 10^4$, $0.6 \times 10^4$, $0.65 \times 10^4$, $0.7 \times 10^4$, $0.75 \times 10^4$, $0.8 \times 10^4$, $0.85 \times 10^4$, $0.9 \times 10^4$, $0.95 \times 10^4$ $M^{-1}s^{-1}$ for human ghrelin. The rate constant ($k_a$) at 25°C may be below $0.5 \times 10^5$ $M^{-1}s^{-1}$, preferably below $0.5 \times 10^5$, $0.45 \times 10^5$, $0.4 \times 10^5$, $0.35 \times 10^5$, $0.3 \times 10^5$, $0.25 \times 10^5$, $0.2 \times 10^5$, $0.15 \times 10^5$, $0.1 \times 10^5$ $M^{-1}s^{-1}$ for human ghrelin.

For example, the dissociation rate constant ($k_d$) at 25°C may be at least $10^{-3}s^{-1}$, preferably at least $1 \times 10^{-3}$, $1.5 \times 10^{-3}$, $2 \times 10^{-3}$, $2.5 \times 10^{-3}$, $3 \times 10^{-3}$, $3.5 \times 10^{-3}$, $4 \times 10^{-3}$, $4.5 \times 10^{-3}$, $5 \times 10^{-3}$ $s^{-1}$ for human ghrelin. The dissociation constant ($K_D$) at 25°C is below $10^{-2}$ $s^{-1}$, preferably below $9.5 \times 10^{-2}$, $9 \times 10^{-2}$, $8.5 \times 10^{-2}$, $8.0 \times 10^{-2}$, $7.5 \times 10^{-2}$, $7 \times 10^{-2}$, $6.5 \times 10^{-2}$, $6 \times 10^{-2}$, $5.5 \times 10^{-2}$ $s^{-1}$ for human ghrelin.

The affinity kinetics can be measured for example by a multi-cycle method on the surface plasmon resonance (SPR) phenomenon on a BIAcore Upgrade instrument (BIAcore, GE Healthcare, Piscataway, NJ). In one example, acyl-ghrelin was diluted 0.5 mg/mL in 10 mM sodium acetate buffer, pH 5.0 (BIAcore) and was covalently coupled on the sensor chip CM5 (BIAcore), using the amine coupling kit (BIAcore). The multi-cycle method may be run for example with five serial dilutions of each patient and control IgG: 0.5, 0.25, 0.125, 0.625 and 0.03125 (mg/mL) including a duplicate of 0.125 mg/mL and a blank (buffer only). During each cycle, 60 μL were injected into the flow conduit of the BIAcore instrument with flow speed 30 μL/min at 25°C and 5 minutes of dissociation. Between injections of each sample, the binding surface was regenerated with 10 mM NaOH. The affinity kinetic data can be analyzed using BiaEvaluation 4.1.1 program (BIAcore). For fitting kinetic data, the Langmuir's 1:1 model may be used and the sample values are corrected by subtracting the blank values resulting from the injection of HBS-EP buffer.

Preferably, the antibody does not modulate ghrelin binding in a ghrelin GHSR1a receptor ligand binding assay. Such assays are known to a person skilled in the art and are commercially available, such as the "ghrelin GHSR1a receptor ligand binding assay" from Cisbio.

Preferably, the antibody does not modulate the $Ca^{2+}$ influx in a cell expressing GHSR1a. Therefore, a Calcium Mobilization Assay may be used which is known to the skilled person.

For said assay, any cell line in which a stable expression of the GHSR1a receptor can be established can be used, corresponding receptor constructs are commercially available (*e.g.,* Genecopia, X0963, Accession code U60179.1). Then, the cells are plated in well-plates and after some time of adaption, for example 24 hours, the cells are loaded with a Fluo-4-AM fluorescent indicator dye (Molecular Probes, Inc., Eugene, OR) for 1 hour in an assay buffer and washed four times in assay buffer. Once Fluo-4-AM fluorescent indicator dye is inside the cell, the fluorescence depends on the $Ca^{2+}$ concentration. Intracellular calcium concentration changes can be therefore measured using a fluorometric imaging plate reader (FLIPR, Molecular Devices, Sunnyvale, CA). The skilled person knows to adapt the conditions depending on the cell line used.

The term "specific binding" or "specifically binds" as used herein refers to the situation in which the antibody, or antigen-binding portion thereof, will not show any significant binding (less than about 10%, 9%, 8%, 7%, 6%, 5%, 4%, 3%, 2% or 1%) to molecules other than its specific binding partner(s), a peptide comprising the antigenic epitope.

Preferably, the antibodies of the invention selectively bind to ghrelin molecules comprising SEQ ID NO: 1 in its acylated or deacylated form and variants thereof as discussed above, and will not bind (or will bind weakly) to non-ghrelin proteins.

The present invention encompasses antibodies, and antigen-binding portions thereof, that specifically bind ghrelin. As discussed above, said antibody should not neutralize a human ghrelin or the biological activity of human ghrelin, whether it be acylated human ghrelin or des-acyl human ghrelin, or both. Activities that should not be inhibited are optionally:

(i) the binding of acylated human ghrelin to receptor GHSR1 a;

(ii) signal transduction prompted by acylated human ghrelin binding GHSR1a;

(iii) binding of des-acyl human ghrelin to a binding partner with which it specifically binds; and/or

(iv) signal transduction prompted by des-acyl human ghrelin binding a binding partner with which it specifically binds.

Specific binding of the antibodies of the present invention to human ghrelin, both acylated and des-acyl forms, permits these molecules to be used as therapeutics or prophylactics for ghrelin-associated diseases and disorders, *i.e.,* diseases or disorders that benefit from increasing a ghrelin bioactivity and therefore increasing the ghrelin pharmacological effect or increasing the level of active ghrelin present in the subject.

Ghrelin and ghrelin-reactive antibodies of the invention may be useful in a method of treatment or prevention of a disease or disorder related to reduced appetite.

By "appetite" is meant the desire to eat food, felt as hunger. Appetite exists in all higher life-forms, and serves to regulate adequate energy intake to maintain metabolic needs. It is regulated by a close interplay between the digestive tract, adipose tissue and the brain. Appetite is assessed in an individual by measuring the amount of food ingested and by assessing the individual's desire to eat. Dysregulation of appetite contributes to anorexia nervosa, bulimia nervosa, cachexia, overeating, and binge eating disorder.

A compound has an "appetite stimulating function" if an individual to whom said compound was administered shows a stimulation of food intake resulting in an increase of food intake.

The composition according to the invention has an appetite stimulating function and stimulates food intake. Due to this function, the composition may be called in one embodiment interchangeably orexigenic or appetite stimulant because orexigenics increase the appetite of an individual.

"Food intake" can be measured using a multitude of techniques including self-reporting using, *e.g.,* diaries or questionnaires, measurements of calorie-intake from a buffet meal, using weighing of food prior to ingestion, or weighing and analysis of paired quantities of food. The food intake may be measured on a meal basis, a daily basis, a weekly basis or a monthly basis. In a preferred embodiment of the invention, the treatment results in a 1% increase in food intake, such as an increase of 2%, more preferably 3% or 5% or 7%, and even more preferred 10% above average food intake prior to initiation of treatment. In another embodiment, the treatment leads to increase in calorie intake irrespective of changes in food intake, since amount of food ingested may not be directly related to the ingested calorie intake, as the various food items such as fat, carbohydrates and proteins, contain different amounts of calories per amount food. In a preferred embodiment of the invention, the treatment results in a at least one 1% increase in calorie intake, such as an increase of 2%, more preferably 3%, or 5% or 7%, and even more preferred 10% in calorie intake.

In one aspect, the present invention relates to stimulation of weight gain or maintaining a stable body-weight by administering the orexigenic composition.

The appetite stimulating function of said composition may be tested in administration experiments by injecting intraperitoneally in free feeding rats either ghrelin alone or together with an anti-ghrelin antibody of the invention. In one embodiment, 1 nmol dose of human ghrelin is chosen for the co-administration experiment aiming at better distinguishing potential modulatory effects of the anti-ghrelin antibodies.

In one example, said anti-ghrelin antibodies are IgG antibodies isolated from individuals suffering from obesity, anorexia nervosa or normal individuals. After administration of the composition or compound, the food intake of the animals is measured. Food intake can be measured for example at 30 minutes to 12 hours, in particular at 3 minutes, 1, 2, 3, 4, 5, 6, 7, 8, 9, 10, 11 and 12 hours.

Reduced appetite or loss of appetite might be due to anorexia, anorexia nervosa, bulimia, cachexia, lipodystrophia or wasting diseases.

Cachexia might be caused by cancer, AIDS, chronic obstructive lung disease, multiple sclerosis, congestive heart failure, tuberculosis, familial amyloid polyneuropathy, mercury poisoning (acrodynia) and hormonal deficiency.

"Anorexia" as used in the context of this invention is the decreased sensation of appetite. While the term in non-scientific publications is often used interchangeably with anorexia nervosa, many possible causes exist for a decreased appetite, some of which may be harmless, while others indicate a serious clinical condition or pose a significant risk. Anorexic individuals according to the invention are defined as individuals having a BMI < 17.5.

"Anorexia nervosa" is an eating disorder characterized by immoderate food restriction and irrational fear of gaining weight, as well as a distorted body self-perception. Due to the fear of gaining weight, people with this disorder restrict the amount of food they consume. This restriction of food intake causes metabolic and hormonal disorders.

"Bulimia" or "bulimia nervosa" is an eating disorder characterized by binge eating and purging, or consuming a large amount of food in a short amount of time followed by an attempt to rid oneself of the food consumed (purging), typically by vomiting, taking a

laxative or diuretic, and/or excessive exercise. Some individuals may tend to alternate between bulimia nervosa and anorexia nervosa.

In one embodiment, it is envisaged that, according to the present invention, the composition can be administered to any individual suffering from weight loss, reduced appetite or even loss of appetite.

In a further embodiment, said individual may be characterized by a BMI < 17.5.

"Cachexia", or wasting, as it may also be called, is seen with several diseases, such as AIDS, cancer, post hip fracture, chronic heart failure, chronic lung disease, such as COLD, COPD, liver cirrhosis, renal failure, and autoimmune diseases such as rheumatoid arthritis and systemic lupus, sepsis and severe infection. Furthermore, wasting is also seen in aging.

The foremost sign of cachexia is weight loss, not only of fatty tissue but also of muscle tissue and even bone. This non-fatty tissue is also known as "lean body mass."

In addition, there is loss of appetite (anorexia), weakness (asthenia), and a drop in hemoglobin level (anemia).

Recent research has revealed that the condition is now regarded as part of the body's reaction to the presence of the underlying disease. Recent research also indicates that, in some cases, tumors themselves produce substances that induce cachexia. Cachexia is found as the terminal state of many different clinical conditions or in chronic diseases such as cancer, infections, AIDS, congestive heart failure, rheumatoid arthritis, tuberculosis, post-hip fracture, cystic fibrosis and Crohn's disease. It can also occur in elderly people who do not have any obvious symptoms of disease. Although cachexia represents the complex metabolic syndrome that is seen in such patients it is commonly recognized as a progressive weight loss with depletion of host reserves of adipose tissue and skeletal muscle.

The core of "cancer cachexia" syndrome relates to the problem of progressive tumor growth and the catabolic side effects of conventional anti-neoplastic therapy. These two phenomena give rise to alterations in the neuro-endocrine system, to the production of a variety of pro-inflammatory cytokines and to the release of cancer specific cachectic factors. In turn, these mediators cause either a reduction in food intake, abnormality in the metabolism or a combination of these two.

Cancer cachexia is reported to occur in about half of all cancer patients and is associated with more than 20 percent of cancer deaths. The condition often occurs during advanced cancer, in particular, when metastatic tumors are present in the body. Cachexia is also more common in children and elderly patients.

Specific cancers are also consistently identified where the frequency of cancer cachexia is particularly high: upper GI cancers (including: pancreas, stomach, oesophagus and liver), Lung cancer, in particular, small cell lung cancer, Head and neck cancer, Colorectal cancer and other solid tumors.

Furthermore, at the moment of diagnosis, 80% of all patients with cancer in upper GI tract and 60% of all patients with lung cancer have already experienced substantial weight loss. On average, the prevalence of cachexia increases from 50 percent to more than 80% percent before death and in more than 20% of the patient's cachexia is the main cause of death.

To detect cancer cachexia, the nutritional state is evaluated with a combination of clinical assessment, anthropometric tests (body weight, skin fold thickness and mid arm circumference) and imaging (DEXA scan, MR scan, CT scan and bioelectric impedance measuring). Cachexia is generally suspected if the involuntary weight loss of greater than 5% of the premorbid weight is observed within a six-month period, especially when combined with muscle wasting.

The most commonly used laboratory parameter is serum albumin. It is however an unspecific parameter. Other markers are proteins with a short half-life transferrin and transthyretin has also been used. Other markers of cachexia are IGF-1, IGFBP-3, ALP (alkaline phosphatase) and testosterone.

Cancer may cause cachexia through a variety of mechanisms, including induction of anorexia and/or increase or change of metabolism.

Energy intake has been shown to be substantially reduced among weight-loosing cancer patients in case of anorexia.

Cancer patients may frequently suffer from physical obstruction of the GI tract, pain, depression, constipation, malabsorption, debility or the side effects of treatment such as opiates, radiotherapy or chemotherapy, which all may decrease food intake. Cancer-associated hypercalcemia may also induce nausea, vomiting and appetite loss. However there remain a large number of patients with cancer in whom there is no obvious clinical cause of reduced food intake.

The central mechanism of cancer-induced anorexia and cachexia is complex and includes many different cytokines, hormones and other factors produced by the cancer cells.

It is envisaged that the composition of the invention may be administered to any individual suffering from any cancer type, regardless of etiology, to successfully treat, reduce or prevent cancer cachexia. Thus, in some embodiments of the present invention, the treatment of an individual with said composition is for the treatment or prevention of cancer cachexia caused by one or more of the following cancer types:

acute lymphoblastic leukemia, acute myeloid leukemia, adrenocortical carcinoma, AIDS-related cancers, AIDS-related lymphoma, anal cancer, childhood cerebellar astrocytoma, childhood cerebral astrocytoma, basal cell carcinoma, extrahepatic bile duct cancer, bladder cancer, bone cancer, osteosarcoma/malignant fibrous histiocytoma, brain stem glioma, brain tumor, breast cancer, male breast cancer, bronchial adenomas/carcinoids, Burkitt's lymphoma, carcinoid tumor, carcinoma of unknown primary, primary central nervous system lymphoma, cerebral astrocytoma/malignant glioma, cervical cancer, childhood cancers, chronic lymphocytic leukemia, chronic myelogenous leukemia, chronic myeloproliferative disorders, colon cancer, cutaneous T-cell lymphoma, endometrial cancer, childhood ependymoma, esophageal cancer, Ewing's family of tumors, childhood extracranial germ cell tumor, extragonadal germ cell tumor, eye cancer, intraocular melanoma eye cancer, retinoblastoma, gallbladder cancer, gastric (stomach) cancer, gastrointestinal carcinoid tumor, gestational trophoblastic tumor, glioma, hairy cell leukemia, head and neck cancer, hepatocellular (liver) cancer, Hodgkin's lymphoma, hypopharyngeal cancer, childhood hypothalamic and visual pathway glioma, intraocular melanoma, islet cell carcinoma (endocrine pancreas), Kaposi's sarcoma, kidney (renal cell) cancer, laryngeal cancer, lip and oral cavity cancer, lung cancer, non-small cell, small cell lung cancer, non-Hodgkin's lymphoma, macroglobulinemia, Waldenström's, malignant fibrous histiocytoma of bone/osteosarcoma, childhood medulloblastoma, melanoma, Merkel cell carcinoma, adult malignant mesothelioma, childhood mesothelioma, metastatic squamous neck cancer with occult primary, multiple endocrine childhood neoplasia syndrome, multiple myeloma/plasma cell neoplasm, mycosis fungoides, myelodysplastic syndromes, myelodysplastic/myeloproliferative diseases, multiple myeloma, chronic myeloproliferative disorders, nasal cavity and paranasal sinus cancer, nasopharyngeal cancer, childhood nasopharyngeal cancer, neuroblastoma, oropharyngeal cancer, ovarian cancer, ovarian epithelial cancer, ovarian germ cell tumor, ovarian low malignant potential tumor, pancreatic cancer, parathyroid cancer, penile cancer, pheochromocytoma, pineoblastoma and supratentorial primitive neuroectodermal tumors, pituitary tumor, pleuropulmonary blastoma, prostate cancer, tinoblastoma, rhabdomyosarcoma, childhood salivary gland cancer, adult-onset soft tissue sarcoma, soft tissue sarcoma, uterine sarcoma, Sezary syndrome, skin cancer (non-melanoma), skin carcinoma, small intestine cancer, testicular cancer, thymoma and thymic carcinoma, thyroid cancer, transitional cell cancer of the renal pelvis and ureter, urethral cancer, endometrial uterine cancer, uterine sarcoma, vaginal cancer, visual pathway and hypothalamic glioma, childhood, Waldenström's macroglobulinemia, Wilms' tumor.

As discussed above, cancer cachexia may be due to a catabolic disorder, i.e., a hypermetabolic state as described above, either resulting from the progressive tumor growth or from the catabolic side effects of the anti-cancer therapy. However, the cancer cachexia may also be due to an anorectic disorder, such as is the case when the individual suffering from the cancer has no appetite or the position of the tumor reduces food intake.

Accordingly, in some embodiments, cachexia is caused by a catabolic disorder. This is particularly suitable when the cancer is a GI tract cancer, especially upper GI tract cancer (it is to be understood herein that the term "upper GI tract cancer" also encompasses pancreatic cancer), lung cancer, in particular, small cell lung cancer, liver cancer (it is to be understood herein that the term "liver cancer" also encompasses metastatic cancer processes in the liver).

In some embodiments, the cancer cachexia is caused by an anorectic disorder.

In some embodiments, cancer cachexia is independent of how the cancer has induced the cachexia, as well as for cachexia caused by a combination of the catabolic disorder and the anorectic disorder.

In some embodiments, cancer cachexia is caused by a solid tumor.

Another sub-group of cancers are those with anorexia caused by dysregulation of the central appetite regulatory center in hypothalamus, where other possible reasons to eat less are excluded. In particular, individuals in terminal cancer states where further cancer treatment is impossible would benefit from a treatment according to the invention as a palliative treatment to increase food intake, improve the digestion and metabolism. Accordingly, a third aspect of the invention relates to the palliative treatment of terminal cancer states in an individual in need thereof, such as wherein said individual is suffering from advanced-stage cancer, preferably terminal cancer.

In some embodiments, cancer cachexia is caused in whole or in part by anti-cancer treatment, such as chemotherapy or radiotherapy or combinations thereof.

The individual treated for cancer cachexia may be elderly, such as 60-120 years old, more preferably 70-120 years old, such as 80-120 years old, for instance 90-120 years old. Equally preferably, said individual is a child, such as from 0-20 years old, for example 0-15 years old, such as 0-10 years old, for example 0-5 years old, such as 0-1 years old, such as a newborn child less than 2 months old.

The composition of the invention may be administered prophylactically for preventing a cachectic state. In this embodiment, the treatment may be started before any antineoplastic treatment initiates. It may be administered continuously during the anti-neoplastic treatment or it may be administered at intervals, for example between periods with anti-neoplastic therapy. By administering during and in particular between the periods of anti-neoplastic therapy, the risk that the treated individual acquires infections and other complications may be reduced due to the better health conditions.

The treatment or prevention of reduced appetite using the composition of the invention may be achieved using any administration method known in the art. Preferably, it may be achieved using any of the administration methods described herein, more preferably using i.v. or subcutaneous administration, most preferably using subcutaneous administration methods.

The compositions of the invention may also be used for the treatment of a lipodystrophic syndrome, or for the manufacture of a medicament for the treatment of a lipodystrophic syndrome. "Lipodystrophic syndromes" or "lipodystrophias" encompass a heterogeneous group of rare disorders characterized by partial or generalized loss of adipose tissue depots. There are several different types of lipodystrophias and the degree of fat loss may vary from very small depressed areas to near complete absence of adipose tissue. Some patients may have only cosmetic problems while others may also have severe metabolic complications such as dyslipidemia, hepatic steatosis, and severe insulin resistance. These disorders can either be inherited (familial or genetic lipodystrophias) or can occur secondary to various types of illnesses or drugs (acquired lipodystrophias).

Inherited lipodystrophias are caused by mutations (alterations or blips) in a gene. Several genes responsible for different types of inherited lipodystrophias have been identified. These include AGPAT2 (1-acylglycerol-3-phosphate O-acyltransferase 2), BSCL2 (Berardinelli-Seip congenital lipodystrophy 2) in congenital generalized lipodystrophy (CGL), lamin A/C (LMNA) gene in familial partial lipodystrophy Dunnigan variety (familial partial lipodystrophy) and PPARG (peroxisome proliferator-activated receptor gamma) gene in familial partial lipodystrophy. Several other candidate genes are currently under investigation for other varieties of inherited lipodystrophias.

Acquired lipodystrophias are: HMRT/HIV-induced lipodystrophy in HIV-infected patients (LD-HIV), acquired generalized lipodystrophy (AGL), acquired partial lipodystrophy (APL) and localized lipodystrophy. Acquired lipodystrophias do not have a direct genetic basis. Rather, many mechanisms may be involved. One such mechanism may be an autoimmune response that destroys normal fat cells.

The metabolic consequences of lipodystrophy are highly important for the general health and the survival. The fact that insulin resistance and the consequent progression to diabetes can result from either obesity or lipodystrophy reflects the crucial role of adipose tissue in carbohydrate and lipid metabolism. In the absence of adequate adipocyte capacity, excess calories cannot be diverted to their normal storage depot; instead they accumulate as increased triglyceride stores in liver, in skeletal and cardiac muscle, and in the pancreatic R cell. This extra-adipose lipid accumulations, through as-yet unclear means, is associated with impaired insulin action and, often, diabetes.

In addition to their passive role as storage depots, normal adipocytes secrete a number of peptides ("adipokines") that may influence insulin sensitivity and/or energy balance (Kahn J C I and TEM 2002). These include potential insulin sensitizers, such as leptin and Acrp30 (also known as adiponectin), and insulin antagonists, including TNF-$\alpha$, IL-6, and possibly resistin. The insulin resistance of lipodystrophy may therefore be the result of disturbed lipid fluxes and/or abnormalities of adipokine secretion.

However, some individuals will still benefit from stimulation of appetite, particularly those individuals for whom a pathological process has led to a lowered appetite, which will naturally lead to an unhealthy weight loss. Thus, in one aspect, the present invention relates to the stimulation of appetite by administering the composition of the invention. The stimulation of appetite may be measured using for instance a visual analog scale for measuring appetite, feeling of hunger or satiety level. In a preferred embodiment of the invention, the stimulation is at least 5% compared to prior to the treatment, such as 7% higher, more preferably 10% higher or even more preferably 15%, 20%, 30%, 40% or 50% higher.

In the context of the invention, the term "treating" or "treatment", as used herein, means reversing, alleviating, inhibiting the progress of, or preventing the disorder or condition to which such term applies, or one or more symptoms of such disorder or condition.

As used herein, the term "patient" or "patient in need thereof" may be intended for a human or non-human mammal suffering from a reduced appetite. In particular, said patient may be a patient who suffers from anorexia, anorexia nervosa, bulimia, cachexia, lipodystrophia or wasting diseases. In another embodiment, the individual suffers from cancer, AIDS, chronic obstructive lung disease, multiple sclerosis, congestive heart failure, tuberculosis, familial amyloid polyneuropathy, mercury poisoning (acrodynia) and hormonal deficiency.

Antibodies of the present invention can be incorporated either alone or together with ghrelin into pharmaceutical compositions suitable for administration to a subject. Such compounds are administered in combination with a pharmaceutically acceptable carrier, diluent, or excipient, in single or multiple doses.

Pharmaceutical compositions can also comprise combinations of antibodies disclosed herein. Such pharmaceutical compositions are designed to be appropriate for the selected mode or route of administration, and pharmaceutically acceptable diluents, carriers, and/or excipients such as dispersing agents, buffers, surfactants, preservatives, solubilizing agents, isotonicity agents, stabilizing agents, and the like are used as appropriate. Such compositions can be designed in accordance with conventional techniques as disclosed, for example, in Gennaro (1995). Remington: The science and practice of pharmacy (19th ed.). Easton, PA: Mack Publishing Company.

Suitable carriers for pharmaceutical compositions include any material which, when combined with an antibody of the present invention or/and optionally ghrelin, retains the molecule's activity and is non-reactive with the subject's immune system. Pharmaceutical compositions according to the invention can be administered to a subject at risk for, or exhibiting, pathologies as described herein using standard administration techniques including oral, parenteral, via inhalation, or topical, including intravenous, intraperitoneal, subcutaneous, pulmonary, transdermal, intramuscular, intranasal, buccal, sublingual, or suppository administration (rectal or vaginal).

Peripheral systemic delivery by intravenous, intraperitoneal, or subcutaneous injection is preferred. Such pharmaceutical compositions preferably contain an "effective amount" or "therapeutically effective amount," or a "prophylactically effective amount," of one or more antibodies of the invention together with ghrelin. An "effective amount" or a "therapeutically effective amount" refers to an amount effective, at dosages and for periods of time necessary, to achieve the desired therapeutic result. An effective amount or a therapeutically effective amount of an antibody and ghrelin can vary according to factors such as the disease state, age, sex, and weight of the individual, and the ability of the antibody or antibody portion with ghrelin to elicit a desired response in the individual. An effective amount or therapeutically effective amount is also one in which any toxic or detrimental effects of the antibody together with ghrelin are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, a prophylactically effective amount will be less than a therapeutically effective amount.

An effective amount or a therapeutically effective amount is at least the minimal dose, but less than a toxic dose, of an active agent which is necessary to impart therapeutic benefit to a subject. Stated another way, such an amount for treating anorexia nervosa, for example, is an amount that induces, ameliorates, or otherwise causes an improvement in the state of the mammal, for example increasing the food intake.

As is well known in the medical arts, dosages for any one subject depend on many factors, including the patient's size, body surface area, age, the particular compound to be administered, sex, time and route of administration, general health, and other drugs being administered concurrently. A typical dose for an antibody or antigen-binding portion thereof of the present invention can be, for example, in the range of from about 0.01 nmol/kg to about 1000 nmol/kg; however, doses below or above this exemplary range are envisioned, especially considering the aforementioned factors. The daily parenteral dosage regimen is about 0.1 nmol/kg to about 100 nmol/kg of total body weight, preferably from about 0.1 nmol/kg to about 20 nmol/kg, more particularly 0.1 nmol/kg to about 10 nmol/kg, in particular 0.1, 0.2, 0.3, 0.4, 0.5, 0.6, 0.7, 0.8, 0.9, 1, 2, 3, 4, 5, 6, 7, 8, 9, 10 nmol/kg body weight. Patient progress can be monitored by periodic assessment, and the dose adjusted accordingly.

The antibody of the invention may be for administration in combination with ghrelin, either sequentially or simultaneously.

Sequential administration indicates that the components are administered at different times or time points, which may nonetheless be overlapping. Simultaneous administration indicates that the components are administered at the same time.

The antibody of the invention may be administered in combination with ghrelin in a ratio of 10:1 (antibody:ghrelin) to 1:10, preferably 1:1 to 1:10, in particular 1:1, 1:2, 1:3, 1:4, 1:5, 1:6, 1:7, 1:8, 1:9, 1:10.

A typical dose for ghrelin, or a derivative, variant or fragment thereof can be, for example, in the range of from about 0.01 nmol/kg to about 1000 nmol/kg; however, doses below or above this exemplary range are envisioned, especially considering the aforementioned factors. The daily parenteral dosage regimen is about 0.1 nmol/kg to about 100 nmol/kg of total body weight, preferably from about 0.1 nmol/kg to about 40 nmol/kg, in particular 0.4, 0.5, 0.6, 0.7, 0.8, 0.9, 1, 2, 3, 4, 5, 6, 7, 8, 9, 10, 11, 12, 14, 16, 18, 20, 25, 30, 35, 40 nmol/kg body weight. Patient progress can be monitored by periodic assessment, and the dose adjusted accordingly.

Patient progress can be monitored by periodic assessment, and the dose adjusted accordingly.

Methods of treatment envisageable using the compositions of the invention also include non-therapeutic methods, for example cosmetic methods or non-therapeutic methods of increasing appetite. For example, the compositions could be used to increase the weight of stock animals or human individuals in non-pathological applications. Preferably, said methods are not methods of treatment of the human or animal body by surgery or therapy.

Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (*i.e.,* "consisting of").

An "Individual" or "subject" is a living animal or human susceptible to a condition.

In one embodiment, said condition is an underweight condition with a BMI < 17.5 as defined herein. In preferred embodiments, the subject is a mammal, including humans and non-human mammals such as dogs, cats, pigs, cows, sheep, goats, horses, rats, and mice. In the most preferred embodiment, the subject is a human.

**Figure legends**

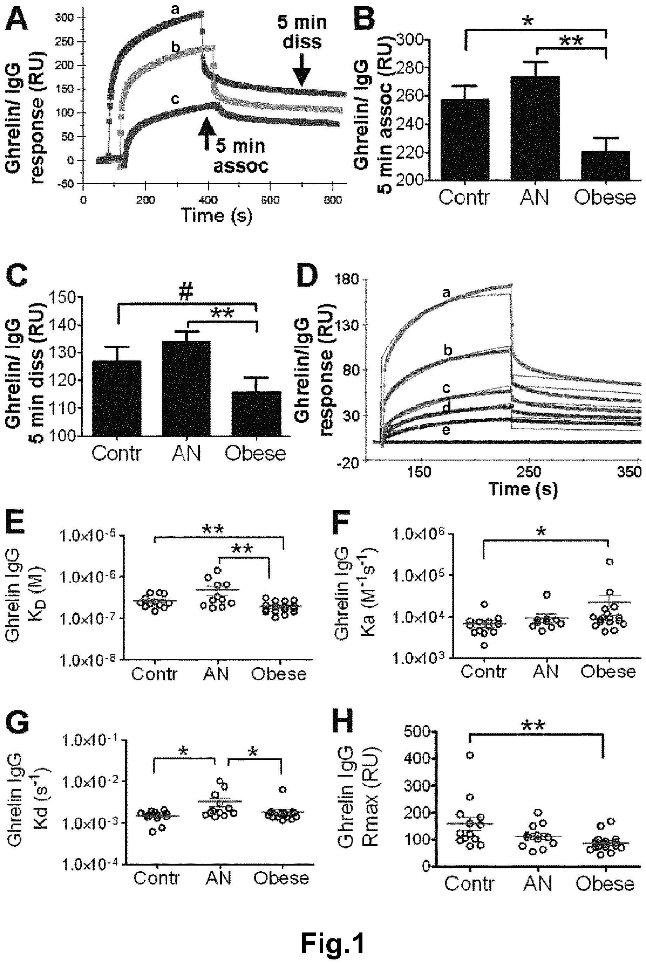

**Figure 1:** Graphs showing the affinity kinetics between ghrelin and IgG from obese, anorectic and control subjects assayed by the SPR technology. (**A**) Representative SPR sensorgrams of association and dissociation to ghrelin of IgG from obese (c), controls (b) and AN patients (a). Mean SPR response in resonance units (RU) between three study groups 5 min after association (**B**) and 5 minutes after dissociation (**C**) as indicated by arrows in **A.** (D) Example of a curve fit for the affinity kinetic analysis using the Langmuir's 1:1 model on sensorgrams obtained from 5 serial dilution of IgG, from top to bottom in mg/mL: 0.5 (a), 0.25 (b), 0.125 (c), 0.625 (d), 0.03125 (e) and blank. This example was taken from a control subject IgG ($k_a$: 9,17x10³ M⁻¹s⁻¹; $k_d$: 1,37x10⁻³ s⁻¹; Rmax: 79,2 RU; $K_D$: 1,50x10⁻⁷ M; Chi²: 21,1). Affinity of IgG of obese was increased comparative to controls and AN patients as represented by lower mean $K_D$ values in obese (**E**). Association ($K_a$) and dissociation ($K_d$) constants are shown in (**F**) and (**G**), respectively, and maximal binding response (Rmax) in (**H**). By applying the bivalent analyte model to fit the same curves, increased mean values of $K_{a1}$ were also found in obese (data not shown). **B:** ANOVA, p=0.002, Tukey's **\*\*p<0.01, \*p<0.05; **C:** K-W test, p=0.009, Dunn's \*\*p<0.01, M-W test, #p<0.05; **E:** K-W test, p= 0,004, Dunn's \*\*p<0.01 AN vs. Obese, Student's T-test \*\*p<0.01 Controls vs. Obese; **F:** K-W test, p= 0,03, Dunn's \*p<0.05; **G:** K-W test, p=0,034, Dunn's \*p<0.05 Controls vs. AN, M-W test, \*p<0.05 AN vs. Obese; **H:** ANOVA p= 0,008, Tukey's \*\*p<0.01. Error bars **B, C, E-H,** s.e.m.

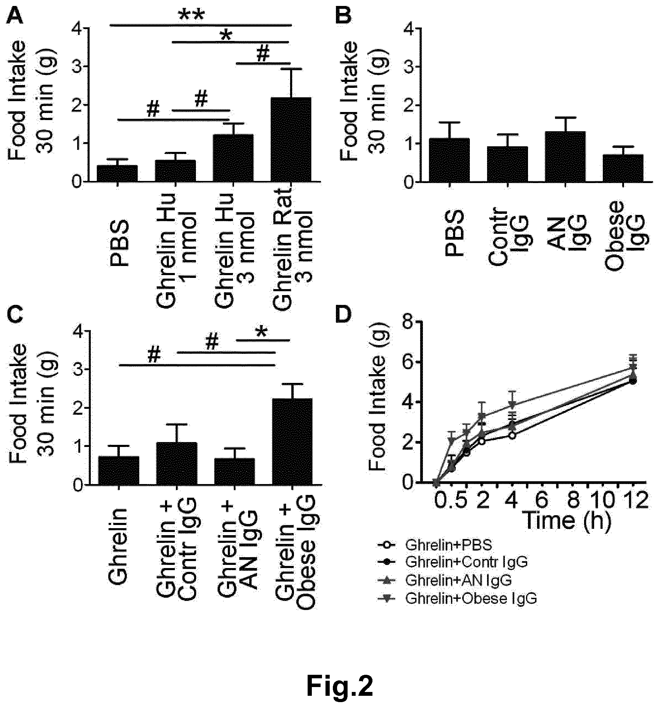

**Figure 2:** Graphs showing the effects of ghrelin-reactive IgG of ghrelin-induced food intake in rats. (**A**) Comparison of orexigenic effects of human (Hu) ghrelin and rat ghrelin in satiated rats. (**B**) No significant differences in food intake were observed in free fed rats after injections of IgG purified from patients and controls. (**C**) Higher 30 min food intake in free fed rats was induced by coadministration of human ghrelin (1 nmol), which alone did not significantly induce food intake as shown in (**A**) together with of IgG purified from plasma of obese (1 nmol), which alone did not significantly change food intake as a shown in (**B**). (**D**) Cumulative food intake in free fed rats measured at 0.5, 1, 2, 4 and 12 hours after injections of ghrelin alone or together with patients' and controls' IgG. **A:** ANOVA, p= 0,005, Tukey's \*\*p<0.01, \*p<0.05, Student's *t*-tests #p<0.05; C: ANOVA, p= 0,031, Tukey's \*p<0.05, Student's *t*-tests #p<0.05. Error bars **A-D,** s.e.m.

## Examples

### Materials and Methods

#### *Study subjects*

Plasma samples were obtained from patients with obesity, anorexia nervosa (AN) and healthy controls. The study was approved by the Ethical Committee of the Kagoshima University, Japan. AN patients were diagnosed according to DSM-IV criteria 26 and were admitted to the Kagoshima University Hospital. All subjects gave written informed consent for study participation. Routine venous blood samples were collected at 8:00 am from all subjects after overnight fast into tubes containing ethylendiaminetetraacetic acid (EDTA, 1 mg/mL) and aprotinin (500 U/mL). The plasma was separated by centrifugation at 4°C and stored at -80°C until assayed.

#### *IgG purification from plasma*

Total IgG were purified from plasma samples that were previously acidified for peptide extraction. The IgG purification was performed using Melon Gel Kit (cat. N. 45206) according to the manufacturer instructions (Thermo Scientific, Pierce, Rockford, USA). IgG containing effluents were saved and frozen at -20°C before lyophilization. Lyophilized IgG were reconstituted in the HBS-EP buffer (BIAcore, GE Healthcare).

#### *Affinity kinetics assay of IgG for ghrelin*

Affinity kinetics of human IgG autoAbs for ghrelin was determined by a biospecific interaction analysis (BIA) based on the surface plasmon resonance (SPR) phenomenon on a BIAcore Upgrade instrument (BIAcore, GE Healthcare, Piscataway, NJ). Acyl-ghrelin (Peptide institute, Inc., Osaka, Japan) was diluted 0.5 mg/mL in 10 mM sodium acetate buffer, pH 5.0 (BIAcore) and was covalently coupled on the sensor chip CM5 (BIAcore), using the amine coupling kit (BIAcore). To compare relative binding response between IgG and ghrelin in obese, AN patients and controls, purified IgG were diluted in each sample to 0.5 mg/mL in HBS-EP buffer (BIAcore) and 25 µL were injected into the flow conduit of BIAcore instrument with flow speed 5 µL/min at 25°C. The values of resonance units (RU) of the sensorgram at were recorded 5 minutes after association and in the end of dissociation 5 minutes after stopping the injection. All samples were analyzed in a random order and alternating injections of IgG from controls, obese and AN patients. For the affinity kinetic analysis, a multi-cycle method was run with five serial dilutions of each patient and control IgG: 0.5, 0.25, 0.125, 0.625 and 0.03125 (mg/mL) including a duplicate of 0.125 mg/mL and a blank (buffer only). During each cycle, 60 µL were injected into the flow conduit of the BIAcore instrument with flow speed 30 µL/min at 25°C and 5 minutes of dissociation. Between injections of each sample, the binding surface was regenerated with 10 mM NaOH. The affinity kinetic data were analyzed using BiaEvaluation 4.1.1 program (BIAcore). For fitting kinetic data, the Langmuir's 1:1 model was used and the sample values were corrected by subtracting the blank values resulting from the injection of HBS-EP buffer. The bivalent analyte model also tested.

*Animals*

Animal care and experimentation were in accordance with guidelines established by the National Institutes of Health, USA and complied with both French and European Community regulations (Official Journal of the European Community L 358, 18/12/1986). Sprague Dawley male rats (n=24), body weight 200-250 g (Janvier, Genest-Saint-Isle, France) were kept in holding cages (3 rats per cage) in a fully equipped animal facility under regulated environmental conditions (22 $\pm$ 1°C, on a 12-hour light-dark cycle with lights on at 7:30 a.m.) for 1 week in order to acclimatize them to the housing conditions. Standard pelleted rodent chow (RM1 diet, SDS, UK) and drinking water were available *ad libitum.* Three days before experiments, the rats were transferred to individual metabolism cages (Tecniplast, Lyon France) where they were fed *ad libitum* with the same RM1 diet but in powdered form (SDS) and drinking water always available. The rats were gently handled daily for several min during acclimation period to habituate them to manipulations associated with intraperitoneal injections and measurements of body weight. At the end of acclimation, rats were distributed into four groups (n=6, in each group) to achieve similar mean body weight. Three food intake experiments were performed in the same rats with 4 days interval between each experiment. All injections were done at 11 a.m. immediately thereafter rats were returned to their metabolism cages, which contained a pre-weighed amount of food. Food intake was measured at 30 minutes, 1, 2, 4 and 12 hours.

*Food intake experiment #1*

To compare effects of human ghrelin and rat ghrelin on food intake in rats, peptides (Peptide institute, Inc., Osaka, Japan) were injected intraperitoneally in 300 $\mu$L of PBS in free fed satiated rats. Human ghrelin was injected with two doses to verify its dose-dependent effect on food intake: 1 nmol/rat (n=6) and 3 nmol/rat (n=6); rat ghrelin was injected with a dose of 3 nmol/rat (n=6); the control group (n=6) received 300 $\mu$L of PBS.

*Food intake experiment #2*

To test effects of human IgG alone on food intake in free feeding rats, four groups of rats (n=6, in each group) received intraperitoneally six different IgG purified from plasma of either obese, or AN or control subjects and control group received PBS. IgG were selected based on their RU values as described in the main text and diluted to 1 nmol in PBS.

*Food intake experiment #3*

To study if IgG from obese, AN or controls can modulate feeding-inducing effect of ghrelin in free fed rats, same rats received intraperitoneally 1 nmol IgG in 300 $\mu$L of PBS as in the Experiment #2 but this time together with 1 nmol of human ghrelin. Each rat injected with IgG received the same IgG as in the previous experiment. The control group that previously was injected with PBS received 1 nmol of human ghrelin only.

*Statistical analysis*

Data were analyzed and the graphs were plotted using the GraphPad Prism 5.02 (GraphPad Software Inc., San Diego, CA, USA). Normality was evaluated by the Kolmogorov-Smirnov test. Group differences were analyzed by the analysis of variance (ANOVA) or the non-parametric Kruskal-Wallis (K-W) test with the Tukey's or Dunn's post-tests, according to the normality results. Where appropriate, individual groups were additionally compared using the Student's *t*-test or the Mann-Whitney (M-W) test according to the normality results. Effects of absorptions were analyzed using the paired *t*-test. Pearson's or Spearman's correlations according to the normality results were analyzed between ghrelin and ghrelin-reactive Ig levels and affinities, and between ghrelin-reactive Ig levels and total Ig concentrations. Data shown as means $\pm$ standard error of means (s.e.m), and for all test, $p < 0.05$ was considered statistically significant.

<u>Results</u>

An ability of a protein to play a carrier role for a small molecule depends on the specificity and reversibility of binding as determined by their affinity kinetics. In this study, SPR technology on a BIAcore instrument was used to determine affinity kinetics between ghrelin and IgG purified from plasma of obese, AN or control subjects using Melon Gel after plasma acidification and peptide extraction. By comparing the binding of each study subject's IgG (0.5 mg/mL) to ghrelin, different dynamics of association and dissociation were found resulting in an overall reduced SPR response in the obese as compared to controls and to AN patients (**Fig. 1A**). A decrease in mean SPR resonance units (RU) values in obese was also recorded at the end of association or after dissociation (**Fig. 1B, C**). Next, the affinity kinetics were analyzed for each study subject using five consecutive IgG dilutions (from 0.5 to 0.03 mg/mL) and the 1:1 Langmuir's fit model (**Fig**. **1D**). In all study subjects, it was found that the equilibrium dissociation constants, $K_D$, values of ghrelin-reactive IgG were in the micromolar range between $10^{-6}$ M and $10^{-7}$ M (**Fig. 1E**). However, the mean level of $K_D$, which is an inverse measure of affinity, in obese subjects was significantly lower than in controls (by a factor 1.2) than in AN patients (by a factor 2.5), for example many $K_D$ values of IgG from obese patients are smaller than $1.7 \times 10^{-7}$ M. This decrease was mainly due to higher association rate constant, $k_a$, values in obese vs. controls (by a factor 3) (**Fig. 1F**), for example many of the association rate constant ($k_a$) values of IgG from obese patients were larger than 10 000 $M^{-1}s^{-1}$, as mean dissociation rate constant, $k_d$, values in obese were not significantly different from controls (**Fig. 1G**). In contrast, increased mean dissociation rate constant $k_d$ values characterized IgG from AN patients (**Fig. 1G**), for example many dissociation rate constant ($k_d$) values of IgG from AN patients were larger than 0.0023 $s^{-1}$. The affinity kinetics in obese subjects' IgG was further characterized by a lower maximal mean SPR response (Rmax), which might reflect their faster saturation (**Fig. 1H**). To determine if affinities of ghrelin-reactive IgG might be relevant to plasma ghrelin concentrations, linear regression analysis was performed. It was found that concentrations of total acyl-ghrelin in obese correlated negatively with $k_a$ but positively with $K_D$ values (Sperman's *r* = -0.5 and *r* = 0.5, respectively, both, *p* = 0.02). Finally, to investigate if IgG may modulate the orexigenic effects of ghrelin, it was injected intraperitoneally in free-feeding rats ghrelin alone or together with human IgG showing different affinities for ghrelin. IgGs derived from 6 different obese with the lowest RU values, IgGs from 6 different AN patients with the highest RU, and IgGs from 6 individuals from the control group which were most similar to the mean RU values were used. As the interaction of human ghrelin with human autoAbs was studied, human ghrelin was also used in the rat study. Because human ghrelin is different from the rat ghrelin by two amino acids, the effect of human ghrelin on food intake in rats was analyzed first. It was found that 3 nmol but not 1 nmol of human ghrelin increased 30 minute-food intake in rats, but it was less potent than rat ghrelin (**Fig. 2A**). Nevertheless, the 1-nmol-dose of human ghrelin selected for the co-administration experiment aiming at better distinguishing potential modulatory effects of IgG on ghrelin-induced food intake; in fact, 1 nmol of rat ghrelin was previously identified as the smallest dose stimulating food intake in satiated rats. It was found that after co-administration of 1 nmol of human ghrelin with 1 nmol of IgG from obese, AN patients or controls, only rats receiving the combination with IgG from obese displayed increased food intake as compared to the other groups (**Fig. 2C**). This orexigenic effect was acute and gradually disappeared by 12 hours after injection (**Fig. 2D**). The enhanced feeding effect associated with obese IgG was linked to their co-administration with ghrelin, as injection of 1 nmol of the same IgG alone had no effects on food intake in free feeding rats at 30 minutes or later (**Fig. 2B**). These data support a carrier role of ghrelin autoAbs which depends on IgG affinity kinetics. Given that the increase in mean $k_a$ was by factor 3 in IgG from obese, one may consider that while IgG from controls will bind to one molecule of ghrelin, IgG from obese will bind and dissociate from three molecules.

The micromolar range of the affinity of ghrelin-reactive IgG found in all study subjects is well positioned to mediate the peptide transport because it will not compete with the nano- to pico-molar affinity of interaction between ghrelin and its receptor. Instead, ghrelin-reactive IgG might protect ghrelin from degradation by hydrolyzing enzymes that deacylate ghrelin, which generally display millimolar affinities for their substrates and, hence, may explain increased ratios of acyl/des-acyl forms of ghrelin in the obese. Thus, a slight increase in affinity of ghrelin-reactive IgG in obesity will improve the autoAbs properties as ghrelin carriers/protectors, while not antagonizing ghrelin receptor binding. In agreement with these data, previous studies have also reported that micromolar affinity is a common feature of natural IgG autoAbs. This is in contrast to high affinity ghrelin neutralizing antibodies, as produced by immunization, which were shown to reduce food intake after their peripheral administration in mice. Moreover, IgG from AN patients displayed a tendency for decreased affinity for ghrelin suggesting that, inversely to obesity, its decrease might diminish ghrelin's orexigenic effect. Taken together, these data reveal that changes in IgG affinity kinetics for ghrelin may enhance hunger signal in obesity and indicate that ghrelin-reactive IgG might represent a new therapeutic target in obesity and anorexia. Further significance of this finding as a general phenomenon intrinsic to peptide signaling can be explored for other peptide hormones.

**Claims**

1. A composition comprising a ghrelin binding antibody or fragment thereof that specifically binds human ghrelin and that:

    i) exhibits an equilibrium dissociation constant ($K_D$) at 25°C in the range from $10^{-7}$ M to $10^{-6}$M for human ghrelin;
    ii) exhibits an association rate constant ($k_a$) at 25°C in the range from $0.5*10^4$ to $0.5*10^5$ $M^{-1}s^{-1}$ for human ghrelin; and
    iii) exhibits a dissociation rate constant ($k_d$) at 25°C in the range from $10^{-3}$ to $10^{-2}$ $s^{-1}$ for human ghrelin;

    wherein said human ghrelin comprises amino acid sequence SEQ ID NO: 1, and wherein said $K_D$, $k_a$, and $k_d$ values are determined by biospecific interaction analysis between said human ghrelin and said ghrelin binding antibody or fragment thereof by surface plasmon resonance.

2. The composition according to claim **1,** wherein said ghrelin binding antibody or fragment thereof that specifically binds human ghrelin further does not inhibit human ghrelin to bind and activate hGHSR1.

3. A composition according to claim **1** or **2** that further comprises ghrelin or a derivative, variant or fragment thereof that retains the ability to bind and activate hGHSR1.

4. A composition according to any one of claims **1** to **3,** wherein the antibody is a fragment selected from the group consisting of Fv, Fab, F(ab')2, Fab', dsFv, (dsFv)2, scFv, sc(Fv)2, diabodies and VHH.

5. A composition according to any one of claims **1** to **4,** wherein the antibody is a polyclonal antibody isolated from an obese individual.

6. A pharmaceutical composition, comprising a composition according to any one of claims **1** to **5** and further comprising a pharmaceutically acceptable carrier, diluent, or excipient.

7. A composition according to any one of claims **1** to **6,** for use as a medicament.

8. A composition according to claim **7,** for use in enhancing ghrelin pharmacological effects, wherein said ghrelin pharmacological effects include orexigenic, adipogenic and somatotrophic properties, increasing food intake and body weight.

9. A composition according to claim **7,** for use in a method of treatment or prevention of a disease or disorder related to reduced appetite.

10. A composition for use according to claim **9,** wherein the disease or disorder is selected from the group consisting of anorexia, anorexia nervosa, bulimia, cachexia, lipodystrophia and wasting diseases.

11. A composition for use according to claim **10,** wherein cachexia is caused by a disease selected from the group consisting of cancer, AIDS, chronic obstructive lung disease, multiple sclerosis, congestive heart failure, tuberculosis, familial amyloid polyneuropathy, mercury poisoning (acrodynia) and hormonal deficiency.

12. An *in vitro* diagnostic method of selecting patients suffering from reduced appetite as likely to respond to a method of treating or preventing reduced appetite comprising administering to said patients a composition according to any one of claims **1** to **6,** wherein said diagnostic method comprises:

> i) isolating antibodies from a blood, plasma or serum sample of the subject;
> ii) measuring the affinity of said antibodies to ghrelin;
> iii) selecting the patient for an appetite stimulating therapy where the dissociation rate constant of at least one of said antibodies is $k_d > 0.0023$ s$^{-1}$.

**Patentansprüche**

1. Zusammensetzung, die einen Ghrelin bindenden Antikörper oder ein Fragment davon umfasst, der bzw. das humanes Ghrelin spezifisch bindet und der bzw. das:

> i) eine Gleichgewichtsdissoziationskonstante ($K_D$) bei 25 °C im Bereich von $10^{-7}$ M bis $10^{-6}$ M für humanes Ghrelin aufweist;
> ii) eine Assoziationskonstante ($k_a$) bei 25 °C im Bereich von $0,5 * 10^4$ bis $0,5 * 10^5$ M$^{-1}$s$^{-1}$ für humanes Ghrelin aufweist; und
> iii) eine Dissoziationskonstante ($k_d$) bei 25 °C im Bereich von $10^{-3}$ bis $10^{-2}$ s$^{-1}$ für humanes Ghrelin aufweist;

wobei das humane Ghrelin eine Aminosäuresequenz SEQ ID Nr. 1 umfasst und wobei die $K_D$-, $k_a$- und $k_d$-Werte durch biospezifische Interaktionsanalyse zwischen dem humanen Ghrelin und dem Ghrelin bindenden Antikörper oder dem Fragment davon durch Oberflächenplasmonresonanz bestimmt werden.

2. Zusammensetzung nach Anspruch **1,** wobei der Ghrelin bindende Antikörper oder das Fragment davon, der bzw. das humanes Ghrelin spezifisch bindet, weiterhin humanes Ghrelin nicht daran hindert, hGHSR1 zu binden und zu aktivieren.

3. Zusammensetzung nach Anspruch **1** oder **2,** die weiterhin Ghrelin oder ein Derivat, eine Variante oder ein Fragment davon umfasst, das bzw. die die Fähigkeit zum Binden und Aktivieren von hGHSR1 bewahrt.

4. Zusammensetzung nach einem der Ansprüche **1** bis **3,** wobei der Antikörper ein Fragment ist, das aus der Gruppe bestehend aus Fv, Fab, F(ab')2, Fab', dsFv, (dsFv)2, scFv, sc(Fv)2, Diabodys und VHH ausgewählt ist.

5. Zusammensetzung nach einem der Ansprüche **1** bis **4,** wobei der Antikörper ein polyklonaler Antikörper ist, der von einer adipösen Person isoliert wurde.

6. Pharmazeutische Zusammensetzung, die eine Zusammensetzung nach einem der Ansprüche **1** bis **5** umfasst und weiterhin einen pharmazeutisch akzeptablen Trägerstoff, ein pharmazeutisch akzeptables Verdünnungsmittel oder einen pharmazeutisch akzeptablen Hilfsstoff umfasst.

7. Zusammensetzung nach einem der Ansprüche **1** bis **6** zur Verwendung als ein Arzneimittel.

8. Zusammensetzung nach Anspruch **7** zur Verwendung beim Verstärken von pharmakologischen Effekten von Ghrelin, wobei die pharmakologischen Effekte von Ghrelin orexigene, adipogenische und somatotrophe Eigenschaften beinhalten, wodurch Nahrungsaufnahme und Körpergewicht erhöht werden.

9. Zusammensetzung nach Anspruch **7** zur Verwendung in einem Verfahren zur Behandlung oder Verhinderung einer Krankheit oder Erkrankung, die mit einem verminderten Appetit zusammenhängt.

10. Zusammensetzung zur Verwendung nach Anspruch **9,** wobei die Krankheit oder Erkrankung aus der Gruppe bestehend aus Anorexie, Magersucht, Bulimie, Kachexie, Lipodystrophie und auszehrenden Krankheiten ausgewählt ist.

**11.** Zusammensetzung zur Verwendung nach Anspruch **10,** wobei Kachexie von einer Krankheit verursacht wird, die aus der Gruppe bestehend aus Krebs, AIDS, chronisch-obstruktiver Lungenerkrankung, multipler Sklerose, kongestiver Herzinsuffizienz, Tuberkulose, familiärer Amyloidpolyneuropathie, Quecksilbervergiftung (Akrodynie) und Hormonmangel ausgewählt ist.

**12.** *In-vitro*-Diagnoseverfahren zur Auswahl von Patienten, die an vermindertem Appetit leiten, als wahrscheinlich auf ein Verfahren zur Behandlung oder Verhinderung von vermindertem Appetit ansprechend, umfassend das Verabreichen einer Zusammensetzung nach einem der Ansprüche **1** bis **6** an die Patienten, wobei das Diagnoseverfahren umfasst:

i) Isolieren von Antikörpern aus einer Blut-, Plasma- oder Serumprobe von dem Probanden;
ii) Messen der Affinität der Antikörper für Ghrelin;
iii) Auswählen des Patienten für eine appetitstimulierende Therapie, wobei die Dissoziationsratenkonstante von mindestens einem der Antikörper $k_d > 0{,}0023$ $s^{-1}$ ist.

## Revendications

**1.** Composition comprenant un anticorps fixant la ghréline ou un fragment de celui-ci qui se lie spécifiquement à la ghréline humaine et qui :

i) présente une constante de dissociation à l'équilibre ($K_D$) à 25°C pour la ghréline humaine dans la gamme de $10^{-7}$ M à $10^{-6}$M ;
ii) présente une constante de vitesse d'association ($k_a$) à 25°C pour la ghréline humaine dans la gamme de $0.5{*}10^4$ à $0.5{*}10^5$ $M^{-1}s^{-1}$ ; et
iii) présente une constante de vitesse de dissociation (kd) à 25°C pour la ghréline humaine dans la gamme de $10^{-3}$ to $10^{-2}$ $s^{-1}$ ;

ladite ghréline humaine comprend la séquence d'acides aminés SEQ ID NO: 1, et les valeurs desdites constantes $K_D$, $k_a$, and $k_d$ sont déterminées par l'analyse d'interaction bio-spécifique, entre ladite ghréline humaine et ledit anticorps fixant la ghréline ou un fragment de celui-ci, par résonance de plasmons de surface.

**2.** Composition selon la revendication **1,** dans laquelle ledit anticorps fixant la ghréline, ou le fragment de celui-ci, se liant spécifiquement à la ghréline humaine, n'inhibe pas la ghréline humaine de se lier et activer hGHSR1.

**3.** Composition selon la revendication **1** ou **2,** qui comprend en outre de la ghréline ou un dérivé, un variant ou un fragment de celle-ci qui conserve la capacité de se lier et d'activer le hGHSR1.

**4.** Composition selon l'une quelconque des revendications **1** à **3,** dans laquelle l'anticorps est un fragment choisi dans le groupe constitué par Fv, Fab, F(ab')2, Fab', dsFv, (dsFv)2, scFv, sc(Fv)2, diabodies et VHH.

**5.** Composition selon l'une quelconque des revendications **1** à **4,** dans laquelle l'anticorps est un anticorps polyclonal isolé à partir d'un individu obèse.

**6.** Composition pharmaceutique comprenant une composition selon l'une quelconque des revendications **1** à **5,** comprenant en outre un véhicule, un diluant ou un excipient pharmaceutiquement acceptable.

**7.** Composition selon l'une quelconque des revendications **1** à **5,** pour son utilisation en tant que médicament.

**8.** Composition selon la revendication **7,** pour son utilisation dans l'amélioration des effets pharmacologiques de la ghréline, lesdits effets pharmacologiques de la ghréline comprenant des propriétés orexigènes, adipogènes et somatotrophiques, augmentant la prise alimentaire et le poids corporel.

**9.** Composition selon la revendication **7,** pour son utilisation dans une méthode de traitement ou de prévention d'une maladie ou d'un trouble lié à une perte d'appétit.

**10.** Composition pour son utilisation selon la revendication **7,** dans laquelle la maladie ou le trouble est choisi dans le groupe constitué par l'anorexie, l'anorexie nerveuse, la boulimie, la cachexie, la lipodystrophie et les maladies débilitantes.

**11.** Composition pour son utilisation selon la revendication **10,** dans laquelle la cachexie est provoquée par une maladie choisie dans le groupe consistant en cancer, SIDA, pneumopathie chronique obstructive, sclérose en plaques, insuffisance cardiaque congestive, tuberculose, polyneuropathie amyloïde familiale, intoxication au mercure (acrodynie et une déficience hormonale.

**12.** Méthode de diagnostic *in vitro* pour sélectionner des patients souffrant d'une perte d'appétit, susceptibles de répondre à une méthode de traitement ou de prévention de la perte d'appétit, ladite méthode de traitement comprenant l'administration auxdits patients d'une composition selon l'une quelconque des revendications **1** à **6,** ladite méthode de diagnostic comprenant :

i) isoler les anticorps d'un échantillon de sang, de plasma ou de sérum du sujet;
ii) mesurer l'affinité desdits anticorps contre la ghréline ;
iii) sélectionner le patient pour une thérapie stimulant l'appétit où la constante de vitesse de dissociation d'au moins l'un desdits anticorps est de $k_d > 0.0023$ s$^{-1}$.

Fig.1

**Fig.2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007099346 A
- EP 2050764 A1
- EP 13305495 A

### Non-patent literature cited in the description

- **WU et al.** *Nature,* 2012, vol. 483 (7391), 594-597
- **DRUCE et al.** *Int J Obes Relat Metab Disord.,* 2005, vol. 29 (9), 1130-1136
- **GUTIERREZ et al.** *Proc. Natl Acad. Sci.,* 2008, vol. 105 (17), 6320-6325
- **ASAKAWA et al.** *Gut,* 2005, vol. 54 (1), 18-24
- **FETISSOV et al.** *Nutrition,* 2008, vol. 24 (4), 348-359
- **TERASHI et al.** *Nutrition,* 2011, vol. 27 (4), 407-413
- **GALLAS ; FETISSOV.** *Peptides,* 2011, vol. 32 (11), 2283-2289
- **ZORRILLA et al.** *Proc Natl Acad Sci U S A.,* 2006, vol. 103 (35), 13226-13231
- **NEEDLEMAN ; WUNSCH.** *J Mol Biol.,* 1970, vol. 48 (3), 443-453
- **BEDNAREK et al.** *J Med Chem.,* 2000, vol. 43 (23), 4370-4376
- **GENNARO.** Remington: The science and practice of pharmacy. Mack Publishing Company, 1995